# FASCICULE DE BREVET EUROPEEN

(11) **EP 2 859 351 B1**
(45) Date de publication et mention de la délivrance du brevet: **28.09.2016**
(21) Numéro de dépôt: 13744806.4
(22) Date de dépôt: 04.06.2013
(51) Int. Cl.: G01N 33/576

(54) **UTILISATION DE TRAF2 ET DE SES HOMOLOGUES POUR DETECTER UNE PROTEINE VIRALE DE CAPSIDE P21 OU UN HOMOLOGUE DE CELLE-CI**
VERWENDUNG VON TRAF2 UND HOMOLOGEN DAVON ZUR ERKENNUNG EINES VIRALEN HÜLLPROTEINS P21 ODER EINES HOMOLOGS DAVON
USE OF TRAF2 AND HOMOLOGUES THEREOF FOR THE DETECTION OF A CAPSIDE P21 VIRAL PROTEIN OR OF A HOMOLOGUE THEREOF

(30) Priorité: 06.06.2012 FR 1255249
(43) Date de publication de la demande: 15.04.2015
(73) Titulaire: Apex Biosolutions, 25000 Besancon (FR); Université de Franche-Comté, 25000 Besançon (FR)
(72) Inventeur: MOROT-BIZOT, Stéphanie, 70190 Sorans-les-Breurey (FR); HERBEIN, Georges, 25320 Montferrand Le Chateau (FR)
(74) Mandataire: Nony
(86) Numéro de dépôt international: PCT/IB2013/054594
(87) Numéro de publication internationale: WO 2013/182987

(56) Documents cités:
- PAWLOTSKY J M: "Virus de l'hépatite C: interactions virus-hôte et diagnostic biologique = Virus-host interactions in hepatitis C virus infection and biological diagnosis", MEDECINE ET MALADIES INFECTIEUSES, SOCIETE FRANCAISE D'EDITIONS MEDICALES, PARIS, FR, vol. 30, no. Suppl. 1, 1 mars 2000 (2000-03-01), pages 14-20, XP009165234, ISSN: 0399-077X, DOI: 10.1016/S0399-077X(00)88856-3
- CHEVALIEZ S ET AL: "Diagnosis and management of chronic viral hepatitis: Antigens, antibodies and viral genomes", BAILLIERE'S BEST PRACTICE AND RESEARCH. CLINICAL GASTROENTEROLOGY, BAILLIERE TINDALL, LONDON, US, vol. 22, no. 6, 1 décembre 2008 (2008-12-01), pages 1031-1048, XP025892677, ISSN: 1521-6918, DOI: 10.1016/J.BPG.2008.11.004 [extrait le 2009-01-31]

## Description

La présente invention se rapporte au domaine du diagnostic biologique.

Plus précisément l'invention concerne l'utilisation d'une protéine Facteur 2 associée au récepteur du Facteur de nécrose tumorale (TNF), TRAF2, ou d'un homologue de celle-ci, comme agent de capture pour la détection d'une protéine virale. L'invention concerne également un procédé et un kit de détection d'une protéine virale.

L'hépatite C est une maladie infectieuse, transmissible par le sang, due au Virus de l'Hépatite C (VHC), et qui se caractérise notamment par une inflammation du foie. Bien que cette infection soit asymptomatique pour 80 % des gens infectés, elle évolue souvent vers une hépatite chronique, puis vers une cirrhose, qui se caractérise par la destruction des cellules du foie, ou hépatocytes, et leur remplacement par du tissu cicatriciel. On parle alors de fibrose cicatricielle du foie.

A terme, une hépatite C peut déboucher sur un cancer du foie, très difficile à traiter dans la mesure où le diagnostic de ce cancer est souvent tardif, et évolue fréquemment vers le décès de l'individu.

Selon les estimations de l'Organisation Mondiale de la Santé (OMS), de 3 à 4 millions de personnes sont infectées chaque année par le VHC. De 130 à 170 millions de personnes ont une infection chronique et se retrouvent donc exposées au risque de développer une cirrhose et/ou un cancer du foie. Chaque année, ce sont plus de 350 000 personnes qui meurent de maladies hépatiques liées au VHC.

Très souvent, la transmission se fait alors que la personne infectée n'a pas été diagnostiquée comme telle. Cela est notamment lié au fait que de nombreuses personnes sont infectées par le VHC sans développer de symptômes. Le virus se transmet par exposition à du sang infectieux, notamment lors d'une transfusion sanguine, d'une transplantation d'organe ou par contamination de l'enfant à la naissance lorsque la mère est infectée. La transmission virale peut également survenir lors de la réutilisation de seringues contaminées, par exemple lors de la consommation de drogues par injection.

Pour des raisons évidentes, il est primordial de pouvoir diagnostiquer de manière précoce, rapide, sensible et à moindre coût, la présence de ce virus chez un individu.

Actuellement, beaucoup d'infections virales, et notamment par le VHC, sont diagnostiquées via la détection d'anticorps dirigés contre l'une des protéines du virus, dans un échantillon biologique, comme le sang, de l'individu testé.

Toutefois, ces tests présentent l'inconvénient de ne pas pouvoir donner un résultat positif avec un bon intervalle de confiance avant une période de 30 à 45 jours à compter de la date présumée de l'infection. Cette période de temps est en effet nécessaire au développement d'une réponse immunitaire suffisante pour être détectable. On parle également de séroconversion. Il est ainsi fréquent que ces tests donnent des résultats faussement négatifs pour des patients infectés peu de temps avant le diagnostic, par exemple moins de 3 semaines. Ils doivent donc être renouvelés une quinzaine de jours après l'obtention d'un résultat négatif afin de confirmer ou d'infirmer celui-ci. Cette seconde analyse génère une augmentation substantielle de coûts.

D'autres tests de diagnostic s'appuient sur la détection de la séquence d'ARN monocaténaire linéaire de ce virus. Bien que de meilleure sensibilité que les tests précédents, ils nécessitent une expertise ainsi qu'un coût très élevés au regard du matériel nécessaire à leur mise en oeuvre.

Depuis plusieurs années maintenant, de nouveaux tests de diagnostic d'une infection par le VHC sont basés sur la détection, dans un échantillon biologique de l'individu testé, de la protéine virale de capside, également appelée p21, ou protéine C, à l'aide d'anticorps spécifiques dirigés contre cette protéine. Bien que ces tests soient une alternative intéressante aux tests détectant la présence de l'ARN du virus, ils n'en restent pas moins d'un coût élevé, dans la mesure où ils nécessitent la mise en oeuvre d'appareillages et d'automates encore chers (Morota et al., J. Virol. Methods, 2009 Apr ; 157(1) : 8-14). Qui plus est, ces tests nécessitent des locaux et une installation spécifiques, ainsi qu'une expertise technique, qui demeurent difficilement accessibles aux pays en voie de développement n'ayant pas les ressources et les infrastructures adéquates à leur mise en place.

Au vu de ce qui précède, il apparaît qu'il demeure un besoin de disposer d'un test de détection d'une infection virale par le VHC qui soit rapide, simple, avec un seuil de sensibilité permettant un diagnostic précoce, et de faible coût.

Il demeure également un besoin de disposer d'un test de détection d'une infection virale par le VHC fiable ne requérant la détection que d'une protéine virale.

Il existe encore un besoin de disposer d'un nouvel outil de détection de protéines virales du VHC qui soit robuste et de bonne sensibilité.

Il existe également un besoin de disposer d'un nouvel outil de capture de protéines virales du VHC.

Il existe également un besoin de disposer d'un test de diagnostic permettant la détection précoce d'une infection virale par le VHC.

La présente invention a précisément pour objet de satisfaire à ces besoins.

Selon un premier objet, la présente invention concerne l'utilisation d'au moins une protéine Facteur 2 associée au récepteur du Facteur de nécrose tumorale, TRAF2, ou d'un homologue de TRAF2, pour la détection ou comme agent de détection d'une protéine virale de capside du Virus de l'Hépatite C, p21, ou d'un homologue de celle-ci.

De manière inattendue, et comme détaillé dans les exemples, les inventeurs ont en effet observé que la protéine TRAF2 peut lier une protéine virale de capside du VHC, ou p21, et former avec elle, au travers d'interactions protéine-protéine directes, un complexe protéique. Ce phénomène permet de favoriser la capture et la concentration de la protéine virale de capside p21, et donc d'améliorer sa détection dans un échantillon biologique, même à de faibles teneurs. Cette propriété peut donc avantageusement être mise à profit pour la capture et la détection de cette protéine virale.

Ainsi, l'invention tire profit d'une propriété nouvellement observée de TRAF2, à savoir sa forte affinité vis-à-vis de cette protéine virale. Cette propriété permet d'abaisser le seuil de détection, avec une fiabilité satisfaisante, de la protéine virale de capside p21 à de très faibles concentrations, par exemple inférieure à 100 pg/ml, voire inférieure à 50 pg/ml, voire encore inférieure à 30 pg/ml.

La présente invention permet donc de réaliser une détection précoce, sensible, rapide, fiable et peu coûteuse d'une infection par le virus de l'hépatite C, exprimant cette protéine p21, ou un homologue de celle-ci. Avantageusement, le diagnostic d'une infection virale au moyen d'une méthode selon l'invention peut ne requérir que la détection de la protéine virale de capside p21, ou d'un homologue de celle-ci.

Selon un autre objet, la présente invention concerne un procédé pour détecter la présence présumée d'une protéine virale de capside du Virus de l'Hépatite C, p21, ou d'un homologue de celle-ci, dans un échantillon biologique, ledit procédé comprenant au moins les étapes consistant à :
a- mettre en contact ledit échantillon avec une quantité efficace d'au moins une protéine Facteur 2 associée au récepteur du Facteur de nécrose tumorale, TRAF2, ou d'un homologue de TRAF2, dans des conditions appropriées à la formation d'au moins un complexe protéique entre la protéine TRAF2 ou l'homologue de TRAF2, et au moins une protéine virale de capside du Virus de l'Hépatite C, p21, ou un homologue de celle-ci,
b- mettre en contact le complexe protéique de l'étape a- avec une entité bi-fonctionnelle, ladite entité étant dotée d'un groupement apte à se lier spécifiquement au moins à une protéine virale de capside p21, ou un homologue de celle-ci, dans ledit complexe, et d'un groupement apte à être détectable, directement ou indirectement, par un mode de détection, la mise en contact étant effectuée dans des conditions appropriées à l'établissement d'une liaison entre ladite protéine virale de capside p21 et ladite entité bi-fonctionnelle,
c- détecter ladite entité bi-fonctionnelle, la détection de ladite entité bi-fonctionnelle liée étant indicatrice de la présence de la protéine virale de capside p21, ou de l'homologue de celle-ci.

Selon un mode de réalisation, une utilisation ou un procédé de l'invention peuvent être mis en oeuvre *in vivo, in vitro* ou *ex vivo,* et de préférence *in vitro* ou *ex vivo.*

Selon encore un autre objet, la présente invention concerne un kit de détection d'au moins une protéine virale de capside du Virus de l'Hépatite C, p21, ou un homologue de celle-ci, comprenant :
- au moins un premier réceptacle comprenant au moins une protéine Facteur 2 associée au récepteur du Facteur de nécrose tumorale, TRAF2, ou un homologue de TRAF2, et
- au moins un second réceptacle comprenant une entité bi-fonctionnelle dotée d'un groupement apte à se lier spécifiquement au moins à ladite protéine virale de capside p21, ou ledit homologue de celle-ci, susceptible d'être présent(e) dans un complexe protéique formé avec la protéine TRAF2, ou l'homologue de TRAF2, et un groupement apte à être détectable, directement ou indirectement, par un mode de détection.

Selon un autre objet, la présente invention concerne un complexe protéique constitué d'une protéine Facteur 2 associée au récepteur du Facteur de nécrose tumorale, TRAF2, ou un homologue de TRAF2, et d'une protéine virale de capside du Virus de l'Hépatite C, p21, ou un homologue de celle-ci.

Selon un autre objet, la présente invention concerne un complexe protéique isolé, constitué d'une protéine Facteur 2 associée au récepteur du Facteur de nécrose tumorale, TRAF2, ou un homologue de TRAF2, et d'une protéine virale de capside du Virus de l'Hépatite C, p21, ou un homologue de celle-ci.

Un tel complexe protéique est avantageusement immobilisé sur un support, notamment tel que défini ci-après dans le présent texte.

Ainsi, la présente invention concerne également un complexe protéique, constitué d'une protéine Facteur 2 associée au récepteur du Facteur de nécrose tumorale, TRAF2, ou un homologue de TRAF2, et d'une protéine virale de capside du Virus de l'Hépatite C, p21, ou un homologue de celle-ci, ledit complexe étant immobilisé sur un support.

Avantageusement, l'invention permet une amélioration de la sensibilité, de la rapidité et de la fiabilité du diagnostic d'infections par le virus de l'hépatite C, exprimant la protéine virale de capside p21, ou un homologue de celle-ci.

Avantageusement encore, l'invention permet de proposer un diagnostic précoce d'une infection par le VHC.

Avantageusement encore, l'invention permet de proposer des tests et des dispositifs pour le diagnostic d'infections par le virus de l'hépatite C, peu coûteux, simples, rapides, et permettant de satisfaire aux différentes exigences dans le domaine, tant sur le plan économique que sur le plan de la santé publique et de la sécurité sanitaire. De tels tests peuvent permettre d'apporter une meilleure garantie concernant les transfusions sanguines humaines, d'établir un diagnostic précoce des infections virales, et d'optimiser les traitements thérapeutiques correspondants.

Au sens de l'invention, par « agent de détection » d'une molécule on entend désigner un composé doté de la propriété de lier spécifiquement, par exemple par affinité, ladite molécule lorsqu'il est mis en contact avec celle-ci dans des conditions appropriées.

Selon l'invention, par « échantillon biologique » on entend désigner tout prélèvement, liquides biologiques, ou tissus ou cellules eucaryotes cultivé(e)s *in vitro* ou *ex vivo,* issus d'un mammifère, notamment humain, et susceptibles d'être infecté(e)(s) par, ou de comprendre, un virus VHC ou une protéine virale de capside p21. Par exemple, un échantillon biologique peut être un prélèvement d'un fluide corporel, tel que le sang, le plasma, les extraits leucocytaires, la salive, l'urine, la bile, les larmes, ou le lait maternel, ou encore un surnageant de milieu de culture, un lysat ou un extrait cellulaire ou tissulaire.

Un tissu biologique en culture peut être un tissu reconstitué *in vitro* ou être un tissu prélevé chez un mammifère et cultivé *ex vivo.*

Les cultures de cellules considérées par l'invention peuvent être des cultures de cellules primaires ou des cultures de lignées cellulaires. Elles peuvent être identiques ou de différentes natures, comme par exemple des cellules dendritiques, des leucocytes, des lymphocytes, des monocytes, des macrophages, etc., infectées ou non par le VHC.

Un mammifère considéré par l'invention peut être tout mammifère susceptible d'être infecté par un virus de l'hépatite C comprenant une protéine virale de capside p21, ou un homologue de celle-ci, tel qu'un primate ou une souris chimère dont le foie contient des hépatocytes humains, et notamment un être humain.

Une « infection virale » peut être toute infection d'une cellule, d'un tissu ou d'un individu, notamment d'un être humain, par un virus possédant et/ou apte à faire produire par la cellule, le tissu ou l'individu infecté(e), une protéine virale de capside p21, ou un homologue de celle-ci, et notamment le virus de l'hépatite C.

Selon l'invention, par « homologue », on entend désigner, à l'égard d'une première séquence d'acides aminés, toute séquence d'acides aminés présentant au moins 80 %, au moins 85 %, au moins 90 %, au moins 95 %, au moins 98 %, au moins 99 % d'homologie avec cette première séquence d'acides aminés, et présentant une activité biologique similaire.

Une homologie de séquence peut être identifiée par toute technique habituellement utilisée dans le domaine par l'homme de l'art. A titre d'exemple, une homologie de séquence peut être déterminée par utilisation de l'interface informatique BLAST disponible sur le site Internet NCBI à l'adresse http://blast.ncbi.nlm.nih.gov configurée avec les paramètres par défaut.

Un homologue d'une première séquence d'acides aminés peut différer de celle-ci, par exemple, par une ou plusieurs délétion(s), et/ou insertion(s) et/ou une ou plusieurs substitution(s) d'un acide aminé. Ces modifications peuvent être combinées. Un homologue d'une première séquence d'acides aminés peut comprendre une ou plusieurs substitutions conservatrices d'acides aminés. Les modifications présentées ci-dessus peuvent être dénommées, de manière générale, « mutation ». Ainsi, les homologues de l'invention concernent également les mutants et les variants de séquences d'acides aminés de l'invention.

A titre d'exemple de mutations que l'on peut considérer dans la présente invention, on peut mentionner le remplacement d'un ou de plusieurs résidus d'acides aminés par des résidus d'acides aminés ayant un indice hydropathique similaire, sans pour autant affecter de manière sensible les propriétés biologiques de la protéine, et notamment sa propriété d'interaction avec sa protéine partenaire. L'indice hydropathique est un indice attribué aux acides aminés en fonction de leur hydrophobicité et de leur charge (Kyte et Doolittle (1982), J. Mol. Biol., 157: 105).

Par "homologue" au sens de l'invention on entend également couvrir des séquences d'acides aminés issues d'une autre espèce que celle retenue pour la première séquence d'acides aminés, mais possédant le degré d'homologie requis et manifestant les propriétés nécessaires à la mise en oeuvre de l'invention.

Les séquences d'acides aminés convenant à l'invention peuvent être naturelles ou synthétiques, le cas échéant susceptibles d'être obtenues par synthèse chimique ou biologique, ou par extraction à partir d'un tissu biologique, exprimant naturellement ou après transduction, la séquence, ainsi que les différentes formes post-traductionnelles, les homologues, les mutants ou les fragments de celle-ci. Elles peuvent être obtenues par tout procédé connu de l'homme de l'art, notamment comme décrit par SAMBROOK et al. (Molecular Cloning : A laboratory Manual, Ed. Cold Spring Harbor, 2001).

Au sens de l'invention, par « environ » on entend indiquer que la valeur qui suit ce terme est vérifiée en prenant en compte les limites d'erreurs expérimentales acceptables pour l'homme du métier.

Au sens de l'invention, « quantité efficace » signifie la quantité minimale et suffisante pour observer la survenue d'un effet recherché, à savoir par exemple la formation d'un complexe comprenant au moins une protéine virale de capside p21, ou un homologue de celle-ci, associée à TRAF2.

### Protéine Facteur 2 associée au récepteur du Facteur de nécrose tumorale (TRAF22)

La protéine Facteur 2 associée au récepteur du Facteur de nécrose tumorale, ou protéine TRAF2, ubiquitaire et codée par le gène TRAF2, est impliquée dans l'une des voies de signalisation initiée par le récepteur du Facteur de nécrose tumorale (TNF).

L'oligomérisation des récepteurs au Facteur de Nécrose Tumorale (TNF) entraine le recrutement de plusieurs protéines au niveau des récepteurs afin de former un complexe protéique constitué en partie de la protéine TRAF2. La formation de ce complexe entraine, via l'activation d'une cascade de protéines kinases dont IKK (Inhibiteur de KappaB Kinase), la phosphorylation de la protéine IkappaB, permettant ainsi la libération de la protéine NF-kappaB. Cette protéine va alors notamment promouvoir, du fait de son passage du cytoplasme vers le noyau des cellules, l'activation de certains gènes cellulaires comprenant, au niveau de leur promoteur, un site de liaison pour NF-kappaB. Cette activation entraîne notamment une augmentation de la prolifération cellulaire et une inhibition de la mort cellulaire programmée, ou apoptose.

Toute protéine TRAF2 peut convenir à l'invention. Une protéine TRAF2 plus particulièrement considérée dans la présente invention est préférentiellement une protéine TRAF2 humaine.

Une protéine TRAF2 selon l'invention peut être obtenue de diverses manières connues de l'homme de l'art. A ce titre, on peut notamment citer l'isolation de ces protéines à partir d'échantillons biologiques humains, qu'il s'agisse d'un prélèvement effectué directement sur un individu ou d'une culture in vitro de cellules humaines, à l'aide de technique d'isolation ou de purification de protéines traditionnellement mises en oeuvre dans les domaines de la recherche ou médical, comme par exemple un western-blot.

Une protéine TRAF2 selon l'invention peut également être obtenue à partir d'une culture bactérienne, telle qu'une culture de bactéries de type *Escherichia coli,* dans lesquelles un gène codant pour une protéine TRAF2, notamment humaine, a été transfecté selon des méthodes de transfection habituellement mis en oeuvre dans le domaine.

A ce titre, on peut par exemple citer l'électroporation qui consiste à appliquer un champ électrique sur les bactéries afin de déstabiliser leurs membranes, permettant ainsi au gène, présent par exemple sur un plasmide, de rentrer dans les cellules où il sera transcrit puis traduit afin de produire une protéine TRAF2. On parle alors de protéine TRAF2 recombinante.

Ainsi, selon un mode de réalisation de l'invention, une protéine TRAF2 conforme à l'invention est préférentiellement une protéine recombinante, et de préférence encore une protéine TRAF2 humaine recombinante.

Une protéine TRAF2 plus particulièrement considérée dans l'invention peut être figurée par la séquence identifiée par la référence SwissProt Q12933 ou un homologue de celle-ci, notamment l'une de ses isoformes de référence SwissProt Q12933-2, Q12933-3 ou Q12933-4, ou par une protéine recombinante.

Une telle protéine TRAF2 recombinante peut notamment être produite dans *Escherichia coli* et peut par exemple être la protéine TRAF2 recombinante commercialisée par la société Proteogenix sous la référence PR-PRO-685-B.

Un homologue de TRAF2 convenant à l'invention présente un degré d'homologie précédemment défini, et une activité biologique similaire. Une activité biologique similaire considérée pour un homologue de TRAF2 peut être une capacité à initier la voie de prolifération cellulaire et/ou à bloquer l'apoptose cellulaire précédemment citée et/ou une aptitude à lier une protéine virale de capside p21 du VHC, ou un homologue de celle-ci, et/ou à lier la partie intracellulaire de récepteurs au TNF.

De préférence, un homologue de TRAF2 convenant à l'invention possède, le cas échéant à un degré différent, ces quatre aptitudes, notamment la capacité à lier une protéine virale de capside p21 du VHC, ou un homologue de celle-ci.

De préférence, une activité biologique considérée pour TRAF2 ou un homologue de celle-ci est son aptitude à lier spécifiquement, par interactions protéine-protéine, une protéine virale de capside p21 du VHC.

### Protéine virale détectable

Une protéine virale détectable au moyen de l'invention est une protéine virale de capside p21 du VHC, ou un homologue de celle-ci. Elle est également appelée protéine C.

Il existe 6 principaux génotypes de ce virus, notés de 1 à 6, et de nombreux sous-types, chacun d'entre eux comprenant la protéine virale de capside p21.

Le VHC est un virus enveloppé de 55 à 65 nm de diamètre à capside icosaédrique renfermant un ARN monocaténaire linéaire de polarité positive. Une fois que cet ARN a pénétré dans la cellule, il sert d'une part d'ARN messager pour la synthèse de protéines virales, et d'autre part de matrice pour la réplication par le complexe de réplication du virus. Cet ARN viral comprend un cadre de lecture ouvert unique comportant de 9024 à 9111 nucléotides qui code pour une polyprotéine précurseur virale unique d'environ 3033 acides aminés qui sera clivée, lors de sa maturation post-transcriptionnelle, de manière à donner les différentes protéines structurelles du virus, dont la protéine virale de capside p21. Cette dernière mesure 191 acides aminés sous sa forme immature et 179 acides aminés sous sa forme mature. Cette protéine sert notamment à la formation de la capside par polymérisation mais est également capable d'interagir avec différents constituants cellulaires.

Parmi ces constituants cellulaires, il a été démontré qu'elle est capable de potentialiser l'activation de NF-kappaB induite par la voie des TNF *via* la voie de signalisation dépendante de TRAF2, en se liant au complexe protéique TNFR1-TRADD-TRAF2 (Chung et al., Biochem. Biophys. Res. Commun. 2001 Jun 1;284(1):15-19) et qu'elle est notamment apte à se lier à la protéine TNFR1 (Zhu et al., J. Virol. 72, 9722-9728).

Selon un mode de réalisation de l'invention, il est possible de mettre en oeuvre une protéine virale p21 de séquence bien déterminée, et en concentration définie, par exemple pour établir un contrôle positif ou une courbe étalon. Une protéine virale de capside p21 pouvant ainsi être mise en oeuvre dans un procédé de l'invention peut être figurée par la séquence identifiée par la référence SwissProt Q01403 (positions 2-191) ou un homologue de celle-ci, ou par une protéine recombinante produite dans *Escherichia coli* et commercialisée par Proteogenix sous la référence PR-HCV-241-A, ou un homologue de celle-ci.

Comme indiqué précédemment, les inventeurs ont observé une interaction spécifique entre une protéine Facteur 2 associée au récepteur du Facteur de nécrose tumorale, TRAF2, ou un homologue de TRAF2, et une protéine virale de capside du Virus de l'Hépatite C, p21, ou un homologue de celle-ci.

La présente invention a également pour objet un complexe protéique constitué de, comprenant, ou consistant en, ces deux protéines.

Avantageusement, ce complexe protéique peut être immobilisé sur un support. Un tel support peut, de préférence, être choisi parmi les supports détaillés ci-après, et de préférence encore être choisi parmi un matériau de cellulose, de nitrocellulose, en plastique, en verre, en céramique, en silice, ou en résine polymérique.

Un complexe protéique de l'invention peut être immobilisé sur un support de manière directe ou indirecte comme développé ci-après.

Selon un mode de réalisation préféré, un complexe protéique est immobilisé sur le support par l'intermédiaire de la protéine TRAF2 ou de son homologue, elle-même étant liée de façon direct au support, ou de manière indirect via une protéine différente de la protéine virale de capside du Virus de l'Hépatite C, p21, ou d'un homologue de celle-ci. Les modes de liaisons d'une protéine TRAF2 à un support peuvent notamment être comme indiqué ci-après.

### Procédé de détection

Un procédé de l'invention pour détecter la présence présumée d'une protéine virale de capside p21, ou d'un homologue de celle-ci, dans un échantillon biologique repose sur la formation d'un complexe protéique entre TRAF2, ou un homologue de celle-ci, et une protéine virale de capside p21, ou l'homologue de celle-ci.

Ainsi, un procédé de l'invention pour détecter, dans un échantillon biologique, la présence présumée d'une protéine virale de capside du Virus de l'Hépatite C, p21, ou d'un homologue de celle-ci, comprend au moins les étapes consistant à :
a- mettre en contact ledit échantillon avec une quantité efficace d'au moins une protéine Facteur 2 associée au récepteur du Facteur de nécrose tumorale, TRAF2, ou d'un homologue de TRAF2, dans des conditions appropriées à la formation d'au moins un complexe protéique entre la protéine TRAF2 ou l'homologue de TRAF2, et au moins une protéine virale de capside du Virus de l'Hépatite C, p21, ou un homologue de celle-ci,
b- mettre en contact le complexe protéique de l'étape a- avec une entité bi-fonctionnelle, ladite entité étant dotée d'un groupement apte à se lier spécifiquement au moins à une protéine virale de capside p21, ou un homologue de celle-ci, dans ledit complexe, et d'un groupement apte à être détectable, directement ou indirectement, par un mode de détection, la mise en contact étant effectuée dans des conditions appropriées à l'établissement d'une liaison entre ladite protéine virale de capside p21 et ladite entité bi-fonctionnelle,
c- détecter ladite entité bi-fonctionnelle, la détection de ladite entité bi-fonctionnelle liée étant indicatrice de la présence de la protéine virale de capside p21, ou de l'homologue de celle-ci.

Avantageusement, TRAF2 peut être immobilisée sur un support comme décrit ci-après.

L'échantillon biologique peut être utilisé brut ou préparé, par exemple par dilution, purification ou ajout de divers réactifs destinés, par exemple, à neutraliser des activités enzymatiques susceptibles d'être délétères pour un procédé de l'invention. L'échantillon biologique est ensuite amené au contact de TRAF2 (étape a-), et laissé incubé un temps suffisant pour la formation du complexe protéique TRAF2-protéine virale de capside p21 (ou un homologue de celles-ci). Un temps d'incubation convenant à l'invention peut varier de quelques minutes, par exemple 10, 20, 30 ou 60 minutes, à quelques heures, par exemple 2, 3 ou 4 heures.

Un procédé de l'invention peut prévoir une étape additionnelle consistant à éliminer le surplus d'échantillon biologique, par exemple par rinçage.

Après formation du complexe, une entité bi-fonctionnelle portant un premier groupe fonctionnel permettant sa liaison avec la protéine virale de capside p21 (ou l'homologue de celle-ci) dans le complexe protéique et un second groupe fonctionnel permettant, directement ou indirectement, sa détection est amené au contact du complexe protéique (étape b-). Le temps d'incubation de l'entité bi-fonctionnelle avec le complexe protéique dépend de l'affinité de l'entité envers la protéine virale de capside p21 (ou un homologue de celle-ci), et peut varier de quelques minutes à quelques heures. Par exemple, le temps d'incubation peut être de 10, 20, 30 ou 60 minutes, ou de 2, 3 ou 4 heures.

Un procédé de l'invention peut comprendre, de préférence, au moins une étape supplémentaire après l'étape b- et avant l'étape c-, ladite étape supplémentaire consistant à éliminer l'entité bi-fonctionnelle non liée à la protéine virale de capside p21, ou à un homologue de celle-ci, à l'étape b-.

Le complexe protéique TRAF2/p21 (ou un homologue de celles-ci) ayant lié l'entité bi-fonctionnelle est ensuite soumis à une étape de détection convenant à la détection du second groupe fonctionnel (étape c-). Celle-ci peut être effectuée directement, si le second groupe fonctionnel est apte par lui-même à émettre un signal détectable, comme un signal luminescent, radioactif ou coloré, ou indirectement si le second groupe doit être mis à en contact et/ou à réagir avec une ou des entité(s) additionnelle(s), telles que des enzymes, des substrats chromogéniques ou luminescents, ou des anticorps eux-mêmes marqués, pour former un ensemble apte à émettre un signal détectable. Le moyen de détection mis en oeuvre est naturellement adapté à la nature du signal à détecter.

### Immobilisation de TRAF2 sur un support

Une protéine TRAF2 (ou un homologue de celle-ci) peut être immobilisée sur tout support habituellement utilisé dans le domaine de la fixation et de la détection des protéines. Un support de l'invention peut être tout matériau solide, souple, flexible ou rigide, sur ou dans lequel TRAF2 (ou un homologue de celle-ci) peut être lié(e), adsorbé(e), ou synthétisé(e) *in-situ.*

Un support convenant à l'invention peut être de nature organique ou inorganique, ou encore être une combinaison de matière organique et inorganique. Avantageusement, un support de l'invention peut être constitué d'un matériau de cellulose, de nitrocellulose, en plastique, en verre, en céramique, en silice, ou en résine polymérique.

Un tel support peut se présenter sous toute forme, adaptée à l'invention. Par exemple, un support de l'invention peut être sous la forme de particules, de gels, de feuilles, de tubes, de sphères, par exemples creuses ou microporeuses, de capillaires, de points, de films, de plaques de puits, de bandelettes, ou de colonnes.

En particulier, un support selon l'invention peut être figuré par une plaque de puits, une bille de latex, une bille de verre, une bille de plastique, une bandelette de papier, cellulose ou nitrocellulose, un microtube, une membrane de nitrocellulose, une membrane de nylon, une puce de silice, une puce recouverte de nanoparticules d'or ou de nanoparticules aimantées, une lame de verre, une surface poreuse, un système microfluidique, ou une nanoparticule aimantée.

De préférence, un support convenant à l'invention peut être une membrane de nitrocellulose ou toute surface convenant à la mise en oeuvre d'un test de type ELISA, telle qu'une plaque en matière plastique comportant des puits.

L'immobilisation de TRAF2, ou un homologue de celle-ci, sur un support peut être effectuée de manière directe par une ou des liaison(s) covalente(s) ou par adsorption. La méthode d'immobilisation de TRAF2, ou un homologue de celle-ci, au support est naturellement choisie de sorte à ne pas affecter les propriétés de celle-ci à lier la protéine virale de capside p21, ou un homologue de celle-ci.

Selon un mode de réalisation préféré, l'immobilisation est effectuée de manière directe par adsorption.

TRAF2 (ou un homologue de celle-ci) peut également être immobilisé sur un support par liaison covalente. Une liaison covalente peut résulter de la réaction d'au moins un des acides aminés de TRAF2 (ou un homologue de celle-ci) ayant ou portant une fonction réactive avec une fonction réactive du support. Une fonction réactive portée par un acide aminé peut, par exemple, être une fonction -SH, -OH, ou -COOH. Ces fonctions peuvent, par exemple, conduire à la formation de ponts disulfure par réaction avec une fonction thiol du support, ou de fonction ester ou amide par réaction respectivement avec une fonction acide, alcool, ou aminée du support.

Les fonctions réactives peuvent être portées naturellement par les acides aminés et/ou le support, ou peuvent avoir été introduites sur les acides aminés et/ou le support, par tout processus de modification connu de l'homme de l'art.

Alternativement, une protéine TRAF2, ou un homologue de celle-ci, peut être immobilisé(e) sur un support de façon indirecte, i.e. par l'intermédiaire d'un espaceur.

Le choix d'un espaceur pour la mise en oeuvre de l'invention sera naturellement effectué de sorte à ne pas altérer l'aptitude de TRAF2, ou un homologue de celle-ci, à lier ou à favoriser la capture de la protéine virale à détecter.

Selon sa nature, un espaceur convenant à l'invention peut être fixé à TRAF2, ou un homologue de celle-ci, par réaction chimique, par génération d'une protéine de fusion TRAF2-espaceur, ou par affinité. L'espaceur peut être fixé au support par tout type d'interactions ou de liaisons appropriées.

Un espaceur peut être une séquence protéique, pouvant de préférence comprendre de 1 à 200 acides aminés, de préférence de 6 à 100 acides aminés, préférentiellement de 10 à 50 acides aminés. Dans un tel mode de réalisation, la liaison TRAF2 - espaceur peut être réalisée par préparation d'une protéine de fusion TRAF2 - espaceur ou par une liaison d'affinité entre TRAF2 et l'espaceur.

Une protéine de fusion TRAF2 - espaceur peut être préparée par production d'une protéine recombinante au moyen de tout outil de biologie moléculaire connu de l'homme de l'art. Une protéine de fusion convenant à l'invention peut être une protéine TRAF2 - polyhistidine. Le groupement polyhistidine peut être lié au support par l'intermédiaire d'ions. Egalement, peut convenir à l'invention une protéine de fusion TRAF2 avec la streptavidine. Cette protéine de fusion peut être immobilisée sur le support par interaction avec la biotine, préalablement fixée au support, par adsorption ou liaison covalente.

De manière préférée encore, un espaceur convenant à l'invention peut être un anticorps spécifique de TRAF2, ou un homologue de celle-ci. L'anticorps peut être lié au support par adsorption.

Une telle mise en oeuvre peut particulièrement convenir au développement de test sous forme ELISA (Enzyme Linked ImmunoSorbent Assay).

### Entité bi-fonctionnelle

Une entité bi-fonctionnelle de l'invention est apte à se lier à au moins une protéine détectable par l'invention dans un complexe tel que défini précédemment.

Une entité bi-fonctionnelle convenant peut être de préférence un anticorps ou un fragment d'anticorps.

L'entité bi-fonctionnelle peut être un anticorps polyclonal ou monoclonal, de type IgG, IgA, IgM, ou IgE. Un anticorps convenant à l'invention peut être choisi parmi des anticorps de souris, de rat, de lapin, de chèvre, de cheval, de lama, d'humain ou d'un autre primate.

Peut également convenir à l'invention un fragment d'anticorps ayant les propriétés de liaison définies ci-dessus. Par « fragment d'anticorps », on entend désigner une portion d'un anticorps telle que Fab, Fab', F(ab)₂, F(ab')₂, et d'autres fragments similaires. Ces termes incluent également toute protéine synthétique ou génétiquement modifiée qui peut agir comme un anticorps en se liant à une protéine détectable de l'invention, dans un complexe protéique tel que défini ci-dessus.

Un anticorps ou un fragment d'anticorps convenant à l'invention peut être préparé par tout procédé connu de l'homme du métier dans le domaine, comme décrit, par exemple, dans « Making and using antibodies : a practical handbook » (Howard & Kaser, Ed. CRC, 2006).

Un anticorps convenant à l'invention peut ne pas être spécifique d'un virus donné, mais peut reconnaitre une famille de protéines virales p21 ou un homologue de celle-ci appartenant à une famille de virus. Alternativement, un anticorps ou un fragment d'anticorps peut être spécifique d'une protéine détectable par l'invention appartenant à une espèce de virus donné. Par exemple, un anticorps ou un fragment d'anticorps peut être spécifique d'une protéine virale de capside p21 du VHC.

A titre d'anticorps anti-protéine p21 du VHC convenant à l'invention, on peut notamment citer les anticorps vendus par la société Proteogenix sous la référence PR-HCV-241-A, les anticorps vendus par la société Santa Cruz sous la référence SC-52855 ou les anticorps vendus par la société Proteogenix sous la référence PR-ANT-286-A.

Une entité bi-fonctionnelle conforme à l'invention comprend un groupement apte à lui conférer la propriété d'être détectable, directement ou indirectement, par un mode de détection. Un groupement détectable peut être propice à une détection luminescente, colorimétrique, ou radioactive, de préférence colorimétrique.

Un groupement détectable « directement » est un groupement apte à générer un signal, par exemple colorimétrique, luminescent, notamment fluorescent, ou radioactif, susceptible d'être détecté directement.

Un groupement luminescent, et en particulier fluorescent, convenant à l'invention peut-être tout marqueur habituellement utilisé dans le domaine par exemple la fluorescéine, le BODIPY, les sondes fluorescentes du type ALEXA, la coumarine et ses dérivés, la phycoerythrine et ses dérivés, ou des protéines fluorescentes telles que la GFP ou le DsRed.

Un groupement radioactif convenant à l'invention peut-être par exemple ³H, ¹²¹I, ¹²³I, le ^{99m}Tc, le ¹⁴C ou ³²P.

Dans le cadre d'un anticorps, un groupe détectable directement peut, par exemple, être lié à la partie Fc.

Un groupement détectable « indirectement » est un groupement nécessitant la présence d'une ou plusieurs entité(s) additionnelle(s), telles que des enzymes, des substrats chromogéniques ou luminescents, ou des anticorps eux-mêmes marqués, pour former un ensemble apte à émettre un signal détectable.

Dans le cadre d'un anticorps, un groupement détectable indirectement peut être une portion Fc nue ou portant un marqueur d'affinité, tel qu'un tag polyhistidine, une biotine, ou une streptavidine, ou portant une enzyme, ou un substrat enzymatique apte à générer un signal par hydrolyse. La détection de ce groupement peut être effectuée, respectivement, par mise en oeuvre d'un anticorps secondaire portant un groupement détectable et apte à reconnaitre la portion Fc ou le marqueur d'affinité, ou par mise en oeuvre, selon le cas, d'un groupe biotine ou d'un groupe streptavidine portant un groupement détectable, ou par mise en oeuvre d'un substrat spécifique de l'enzyme et apte à émettre un signal détectable par hydrolyse, ou par mise en oeuvre d'une enzyme spécifique du substrat.

A titre d'anticorps secondaires convenant à l'invention, on peut notamment citer les anticorps de chèvre anti IgG de souris marqués à l'alcaline phosphatase vendus par la société Santa Cruz sous la référence SC-2008 ou les anticorps de chèvre anti IgG de souris marqués à l'Horse Radish Peroxydase vendus par la société Santa Cruz sous la référence SC-2005.

Selon un mode de réalisation préférée, un procédé de l'invention peut mettre en oeuvre un anticorps anti-protéine virale portant un groupement fonctionnel détectable directement ou indirectement, comme défini précédemment.

Avantageusement, un procédé de l'invention comprenant la mise en oeuvre d'une entité bi-fonctionnelle comprenant un groupe fonctionnel détectable indirectement peut comprendre la mise en oeuvre d'un anticorps portant un substrat enzymatique chromogénique ou une enzyme apte à hydrolyser le substrat enzymatique chromogénique, ou peut comprendre la mise en oeuvre d'un couple anticorps primaire-anticorps secondaire. L'anticorps primaire peut comprendre une portion Fc nue ou portant un marqueur reconnu par l'anticorps secondaire. L'anticorps secondaire peut comprendre un groupe détectable directement comme défini précédemment ou peut comprendre un substrat enzymatique chromogénique ou une enzyme apte à hydrolyser le substrat enzymatique chromogénique.

Selon un mode de réalisation particulier, une enzyme convenant à l'invention peut être une phosphatase alcaline, une tyrosinase, une peroxydase, ou une glucosidase.

Il relève des connaissances de l'homme du métier de déterminer le substrat chromogénique convenant selon l'enzyme considérée.

Lorsque l'enzyme est une phosphatase alcaline, un substrat chromogénique convenant à l'invention peut alors être le BCIP/MBT (5-bromo-4-chloro-3'-indolyphosphate p-toluidine salt - nitro-blue tetrazolium chloride).

Un groupement détectable convenant à l'invention peut être fixé par toutes méthodes appropriées au niveau de l'entité bi-fonctionnelle. Sa localisation sur l'entité bi-fonctionnelle est choisie de manière à ne pas affecter l'aptitude de celle-ci à se lier à une protéine virale de capside p21 (ou à un homologue de celle-ci) dans un complexe protéique de l'invention.

### Mode de détection

Le mode de détection d'une entité bi-fonctionnelle liée à un complexe de l'invention sera naturellement adapté au groupement détectable mis en oeuvre.

Par « mode de détection », on entend tout système capable de détecter l'émission d'un signal par une entité bi-fonctionnelle de l'invention.

Ainsi, un mode de détection de l'invention peut mettre en oeuvre, par exemple, un système de détection en colorimétrie visible, tel que l'oeil nu, un système de détection par radiochimie ou par médecine nucléaire, tel que la scintigraphie, un système de détection par imagerie, par résonance (IRM), par rayons X, par diffusion de lumière, par spectrométrie de masse, par spectroscopie par exemple par fluorescence ou par UV-visible, par ultrasons, par radioactivité, par réfractométrie, optique, piézoélectrique, acoustique, par électrochimie, par conductivité, par pHmétrie ou encore par voie biologique.

Selon un mode de réalisation, un mode de détection plus particulièrement considéré par l'invention s'appuie sur un système de détection en colorimétrie visible, plus particulièrement à l'oeil nu.

La détection peut se faire de manière quantitative ou qualitative.

Une détection qualitative peut conduire à conclure simplement à la présence ou à l'absence de la protéine virale cherchée. Une détection quantitative peut permettre un dosage de la protéine recherchée dans l'échantillon biologique testé.

La quantification du signal obtenu peut-être effectué par comparaison de son intensité à celui obtenu avec un échantillon contrôle dénué de la protéine virale recherchée, et, le cas échéant, à celui obtenu avec un échantillon témoin comprenant la protéine virale cherchée à une concentration connue.

De manière préférée, il est possible de réaliser une série d'échantillons témoins de concentrations connues, et croissantes, de la protéine virale cherchée de sorte à établir une courbe étalon à l'égard de laquelle l'intensité du signal mesuré dans l'échantillon à tester sera comparée. Les valeurs de référence de l'échantillon contrôle et des échantillons témoins peuvent être déterminées simultanément à la mesure de l'échantillon test ou être déterminées antérieurement ou postérieurement. Lorsque les valeurs de référence sont déterminées antérieurement, celles-ci peuvent être stockées dans une base de données et être utilisées pour une série de mesures d'échantillons tests.

### Kit

La présente invention concerne également un kit de détection d'au moins une protéine virale de capside du Virus de l'Hépatite C, p21, ou un homologue de celle-ci, comprenant:
- au moins une protéine Facteur 2 associée au récepteur du Facteur de nécrose tumorale, TRAF2, ou un homologue de TRAF2, et
- au moins un second réceptacle comprenant une entité bi-fonctionnelle dotée d'un groupement apte à se lier spécifiquement au moins à ladite protéine virale de capside p21, ou ledit homologue de celle-ci, susceptible d'être présent(e) dans un complexe protéique formé avec la protéine TRAF2, ou l'homologue de TRAF2, et un groupement apte à être détectable, directement ou indirectement, par un mode de détection.

Un kit de l'invention peut en outre comprendre au moins un réceptacle comprenant une protéine virale de capside p21, ou un homologue de celle-ci, en quantité ou concentration connue. Ce mode de réalisation peut permettre la réalisation d'un contrôle positif ou d'une courbe étalon en protéine virale de capside p21 (ou un homologue de celle-ci).

Un kit de l'invention peut comprendre en outre au moins une instruction de mise en oeuvre de TRAF2, ou d'un homologue de celui-ci, et de l'entité bi-fonctionnelle pour détecter la présence présumée d'une protéine virale de capside p21 (ou d'un homologue de celle-ci) dans un échantillon biologique.

TRAF2, l'entité bi-fonctionnelle, la protéine virale de capside p21, et leurs homologues sont avantageusement tels que définis précédemment.

Selon un aspect de l'invention, TRAF2 peut être utilisée comme agent de capture d'une protéine virale de capside p21 ou de l'un de ses homologues. Cet aspect de l'invention peut être mis en oeuvre à des fins de purification d'un échantillon biologique, ou d'enrichissement d'un échantillon en protéine virale de capside p21.

Dans une telle mise en oeuvre, l'utilisation d'une entité bi-fonctionnelle telle que définie précédemment n'est pas nécessaire.

Après formation d'un complexe protéique TRAF2/p21 (ou homologue de celle-ci), une étape de précipitation ou de séparation du complexe obtenu peut être effectuée, par tout moyen connu de l'homme de l'art. Le complexe est ensuite dissocié pour récupérer la protéine virale. L'étape de dissociation peut-être effectuée par toute méthode connue dans le domaine, tel quel l'utilisation d'un tampon salin adéquat ou une technique d'acidification, et de préférence une technique d'acidification.

Avantageusement, ce mode de réalisation peut être effectué par mise en oeuvre d'une colonne de chromatographie fonctionnalisée au moyen de TRAF2 ou de l'un de ses homologues.

Dans la description ci-avant, l'expression « compris entre » ou « allant de » doit s'entendre bornes incluses.

Les exemples ci-après sont donnés uniquement à titre illustratif de l'invention.

### LEGENDES DES FIGURES

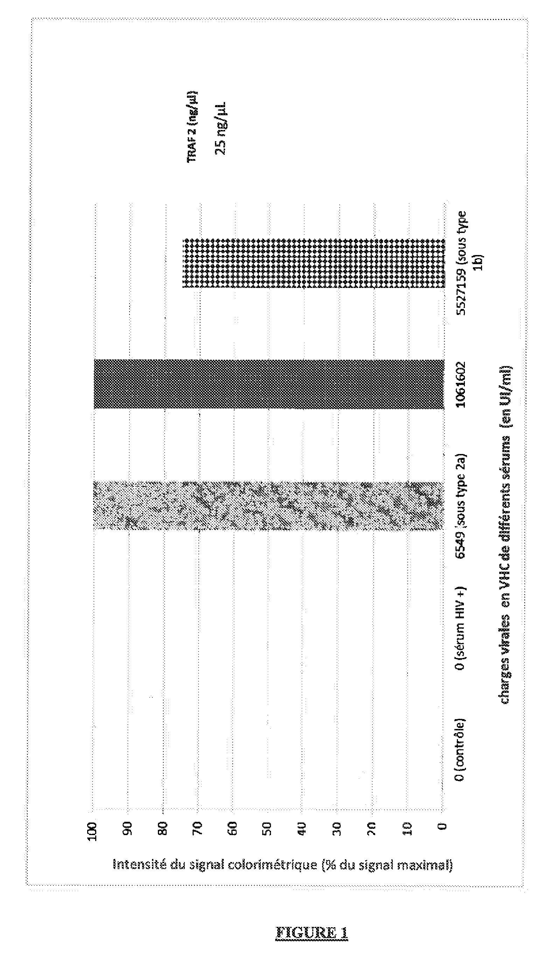

**Figure 1** : illustre les variations de l'intensité du signal colorimétrique, en % du signal maximal obtenu par hydrolyse du substrat BCIP-NBT par la phosphatase alcaline de l'anticorps secondaire fixé à l'anticorps primaire de souris anti-protéine virale de capside du VHC lié au complexe protéine TRAF2 / protéine virale de capside du VHC, en fonction de la concentration en protéines virales de capside du VHC (ou charge virale) de sérums humains infectés par différents types de Virus de l'hépatite C.

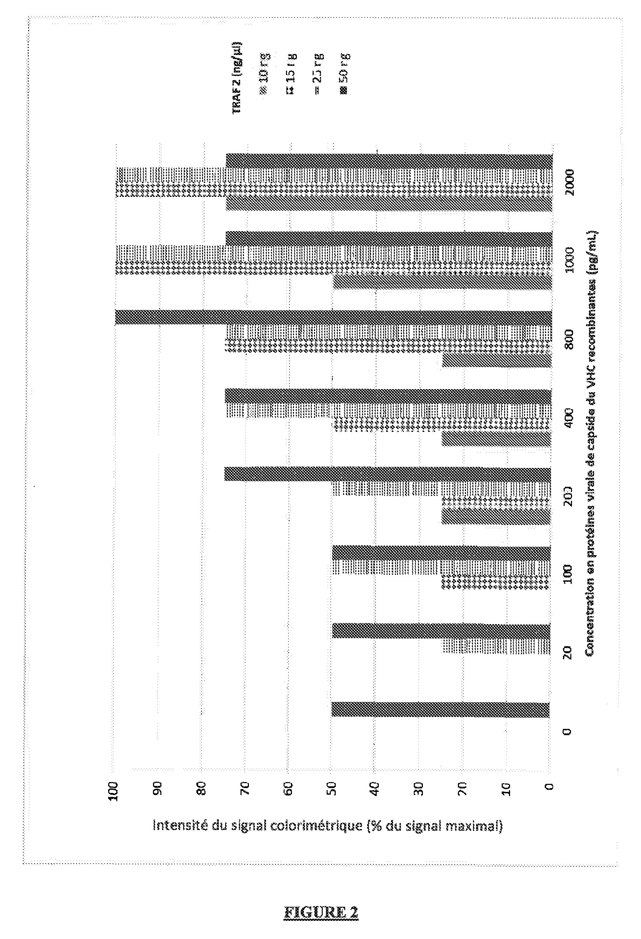

**Figure 2** : illustre les variations de l'intensité du signal colorimétrique, en % du signal maximal résultant de l'hydrolyse du substrat BCIP-NBT par la phosphatase alcaline de l'anticorps secondaire fixé à l'anticorps primaire de souris anti-protéine virale de capside du VHC recombinante fixé au complexe protéine TRAF2 / protéine virale de capside du VHC recombinante, en fonction de la concentration en protéines virales de capside du VHC recombinantes. TRAF2 est mise en oeuvre à raison de 10 ng/µL, 15 ng/µL, 25 ng/µL et 50 ng/µL.

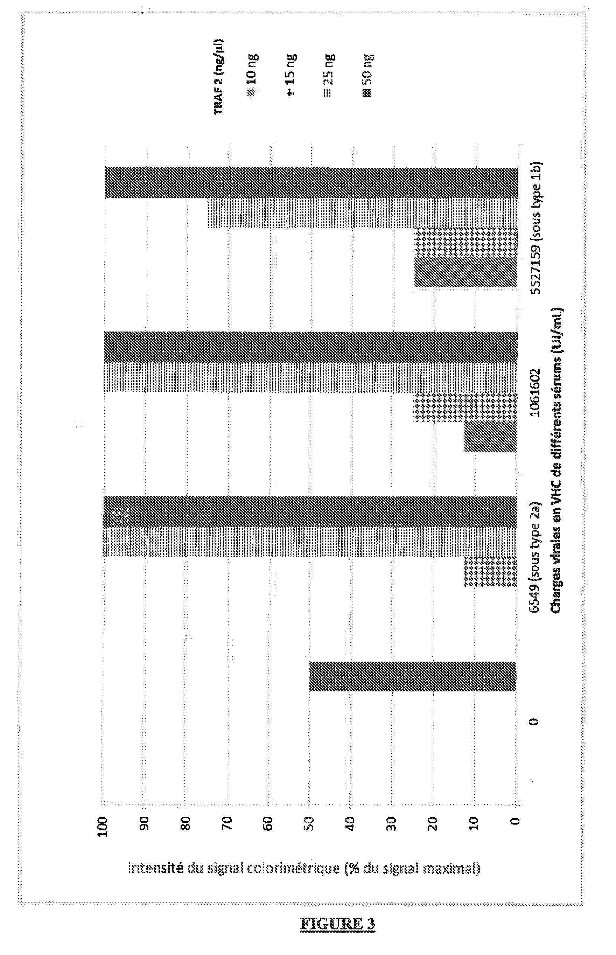

**Figure 3** : illustre la variation de l'intensité du signal colorimétrique en % du signal maximal obtenu lors de l'expérience, résultant de l'hydrolyse de BCIP-NBT par la phosphatase alcaline d'un anticorps secondaires fixé à l'anticorps primaire de souris anti-protéine virale de capside du VHC, lui-même fixé au complexe protéine TRAF2 / protéine virale de capside du VHC, en fonction de la concentration en protéine virale de capside du VHC (ou charge virale) de sérums humains infectés par différents types de Virus de l'hépatite C et en fonction de la concentration en protéine TRAF2 mise en oeuvre à raison de 10 ng/µL, 15 ng/µL, 25 ng/µL et 50 ng/µL.

### EXEMPLES

### Exemple 1

### Interaction entre une protéine Facteur 2 associée au récepteur du Facteur de nécrose tumorale (TRAF2) et une protéine virale recombinante de capside du Virus de l'Hépatite C (p21)

La protéine Facteur 2 associée au récepteur du Facteur de nécrose tumorale, TRAF2, est adsorbée à la surface d'une membrane de nitrocellulose par dépôt d'1 µL d'une solution comprenant 25 ng/µl de protéine TRAF2 (PROTEOGENIX, référence PR-PRO-685-B). Les dépôts, ou spots, sont effectués en ligne sur la membrane et espacés chacun d'environ 1 cm.

La membrane est laissée sécher, et les sites de liaison non spécifiques de la membrane sont saturés par incubation pendant 1 heure dans une solution de saturation (1,5 % de lait écrémé dans du Tris Buffer Saline-0,05 % Tween 20 (TBS-T), vendu par la société Sigma-Aldrich), à température ambiante.

Des échantillons (1 µL) de protéine recombinante comprenant chacun une concentration donnée de protéine virale de capside p21 recombinante (PROTEOGENIX, référence PR-HCV-241-A), à, respectivement, 20 pg/ml, 200 pg/ml, 1000 pg/ml et 2000 pg/ml sont déposés sur la membrane de nitrocellulose. Chaque échantillon est déposé sur un spot différent.

La membrane est rincée puis 1 µL d'une solution d'anticorps primaire de souris anti-protéine virale de capside du VHC, diluée au 1/50^{ème} (SANTA CRUZ, SC - 52855), est disposé sur chaque spot formé à l'étape précédente.

La membrane est rincée dans du TBS-T puis mise à incuber pendant 30 minutes à température ambiante dans une solution d'anticorps secondaire de chèvre anti-anticorps de souris marqué à l'alcaline phosphatase dilué au 1/2500^{ème} (SANTACRUZ SC - 2008).

La membrane est à nouveau rincée dans une solution de TBS-T, puis dans une solution de TBS (Tris Buffer Saline).

La membrane est ensuite laissée à incuber dans une solution de BCIP/NBT (5-bromo-4-chloro-3'-indolyphosphate p-toluidine salt-nitro-blue tetrazolium chloride) (Sigma-Aldrich) jusqu'à apparition de la coloration, environ 5 à 15 minutes.

Enfin, la membrane est rincée à l'eau distillée puis l'intensité de la coloration des différents spots est lue qualitativement à l'oeil nue.

Les résultats sont exprimés en pourcentage d'intensité par rapport au signal maximum obtenu dans l'expérience. Une valeur correspondant à 25 % du signal maximal est retenue comme indiquant positivement la présence de la protéine virale de capside p21.

Les résultats obtenus présentés Figure 1 montrent, d'une part, que la protéine TRAF2 et la protéine virale de capside du VHC p21 interagissent l'une avec l'autre. Le complexe formé peut être détecté avec une bonne sensibilité, à de faibles concentrations en protéine virale de capside p21 (25 % du signal maximal à 20 pg/ml). Cette bonne sensibilité de détection permet avantageusement de diagnostiquer de façon précoce une infection chez un individu par un virus exprimant la protéine virale de capside p21 du Virus de l'Hépatite C.

### Exemple 2

### Tests de détection de l'infection de différents sérums humains par différents types de Virus de l'hépatite C grâce à la mise en évidence de l'interaction entre une protéine Facteur 2 associée au récepteur du Facteur de nécrose tumorale (TRAF2) et une protéine virale de capside du Virus de l'Hépatite C (p21)

Un protocole similaire à celui mis en oeuvre dans l'exemple 1 a été effectué pour ce tests, à ceci près toutefois que les échantillons de protéine recombinante comprenant chacun une concentration donnée de protéine virale de capside p21 recombinante mise en oeuvre dans l'exemple 1 sont remplacés par une même quantité d'un sérum humain :
(i) négatif pour le VHC (contrôle négatif) ;
(ii) positif pour le VHC de génotype 2a et de charge virale en copie d'ARN égale à 6549 UI/mL ;
(iii) positif pour le VHC de génotype 1b et de charge virale en copies d'ARN égale à 5527159 UI/mL ;
(iv) positif pour le VHC (génotype non déterminé) et de charge virale inconnue ;
(v) positif pour le VHC (génotype non déterminé) et de charge virale en copies d'ARN égale à 1061602 UI/mL ;
(vi) négatif pour le VHC mais positif pour le VIH ; ou
(vii) d'un sérum physiologique à titre de blanc (contrôle négatif).

A l'issu de ce test, une coloration visible à l'oeil nu apparaît au niveau des spots sur lequel des sérums humains positifs pour le VHC ont été déposés.

Aucune coloration n'apparaît toutefois au niveau des spots des deux contrôles négatifs ainsi qu'au niveau du spot sur lequel le sérum négatif pour le VHC mais positif pour le VIH a été déposé.

La Figure 1 illustre les résultats obtenus pour les conditions figurées par les échantillons (i), (ii), (iii), (v) et (vi).

Ces résultats démontrent à la fois que le présent test est efficace pour différents types de VHC mais également qu'il est spécifique au VHC, dans la mesure où il ne réagit pas lorsque en présente de sérum issu d'individus infectés par le VIH et non par le VHC.

De plus, la mesure de l'intensité des spots permet d'obtenir un résultat à la fois qualitatif, de par l'apparition d'une coloration, mais également quantitatif. En effet, l'intensité de coloration obtenue au niveau des spots varie en fonction de la charge virale du sérum testé.

Ainsi, l'obtention d'une intensité de coloration pour des sérums dont la charge virale est connue permet de mettre en place une courbe d'étalonnage de la charge virale en fonction de l'intensité des spots. Il est alors possible, lorsqu'un sérum s'avère positif, de mesurer sa charge virale au regard de cette échelle.

Un test similaire dans lequel les sérums ont toutefois été préalablement dénaturés à l'aide d'acide (HCl 0,5M) a également été réalisé. Les résultats obtenus sont identiques à ceux indiqués précédemment.

Ainsi, un test conforme à l'invention tel qu'indiqué précédemment reste efficace même lorsque les sérums subissent un traitement dénaturant par l'acide.

### Exemple 3

### Mise en évidence de la variation de l'intensité du signal colorimétrique des spots, en pourcentage du signal maximal, en fonction de la quantité de TRAF2 et de la quantité de protéines virales recombinantes de capside du Virus de l'Hépatite C (p21)

Un protocole similaire à celui mis en oeuvre dans l'exemple 1 a été effectué. Toutefois, les échantillons (1 µL) de protéine recombinante testés comprennent chacun une concentration donnée de protéine virale de capside p21 recombinante (PROTEOGENIX, référence PR-HCV-241-A), à savoir, respectivement, 20 pg/ml, 100 pg/ml, 200 pg/ml, 400 pg/ml, 800 pg/ml, 1000 pg/ml et 2000 pg/ml. Chaque échantillon est déposé la membrane de nitrocellulose sur un spot différent.

De même, des solutions comprenant 10 ng/µl, 15 ng/µl, 25 ng/µl de protéine *TRAF2 (PROTEOGENIX, référence PR-PRO-685-B)* ont été mises en oeuvre avec les différents échantillons de protéine virale de capside p21 recombinante indiqués ci-dessus.

Les résultats obtenus sont présentés dans le Tableau 1 ci-après ainsi que sur la Figure 2.

| | **Quantité TRAF2 (ng/µl)** | | | |
|---|---|---|---|---|
| **HCV p21 (pg/ml)** | **10** | **15** | **25** | **50** |
| **0** | 0 | 0 | 0 | 50 |
| **20** | 0 | 0 | 25 | 50 |
| **100** | 0 | 25 | 50 | 50 |
| **200** | 25 | 25 | 50 | 75 |
| **400** | 25 | 50 | 75 | 75 |
| **800** | 25 | 75 | 75 | 100 |
| **1000** | 50 | 100 | 100 | 75 |
| **2000** | 75 | 100 | 100 | 75 |

Les résultats sont exprimés en pourcentage d'intensité par rapport au signal maximum obtenu dans l'expérience. Une valeur correspondant à 25 % du signal maximal est retenue comme indiquant de manière positive la présence de protéine virale de capside p21.

Il ressort de ces résultats que le procédé mis en oeuvre dans la présente invention améliore le seuil de détection, même avec une faible quantité de TRAF2 (10 ng/µl).

La quantité optimale en protéine TRAF2 permettant d'obtenir une détection très sensible de la présence de la protéine virale de capside du VHC (dès 20 pg/ml) tout en ne produisant pas de faux positifs (intensité du spot différent de 0 % du signal maximal en l'absence de protéine virale de capside du VHC) est de 25 ng/µl.

### Exemple 4

### Mise en évidence de la variation de l 'intensité du signal colorimétrique des spots, en pourcentage du signal maximal, en fonction de la quantité de TRAF2 et de la charge virale en Virus de l'Hépatite C (p21) dans différents sérums humains,

Un protocole similaire à celui indiqué dans l'exemple 3 ci-dessus a été mis en oeuvre en remplaçant toutefois les échantillons de protéine virale de capside p21 recombinante par une même quantité de trois sérums humains positifs pour le VHC, comprenant différentes charges virales en copie d'ARN, à savoir :
(i) égale à 6549 UI/mL ;
(ii) égale à 1061602 UI/mL ; et
(iii) égale à 5527159 UI/mL.

Les résultats obtenus sont présentés dans le Tableau 2 ci-après ainsi que sur la Figure 3.

| | **Quantité TRAF2** (**ng**/µ**l**) | | | |
|---|---|---|---|---|
| **Charge virale (UI/ml)** | **10** | **15** | **25** | **50** |
| **0** | 0 | 0 | 0 | 50 |
| **6549** | 0 | 12,5 | 100 | 100 |
| **1061602** | 12,5 | 25 | 100 | 100 |
| **5527159** | 25 | 25 | 75 | 100 |

Les résultats sont exprimés en pourcentage d'intensité par rapport au signal maximum obtenu dans l'expérience. Une valeur correspondant à 25 % du signal maximal est retenue comme indiquant de manière positive la présence de protéine virale de capside p21.

Il ressort de ces résultats que, conformément à ce qui a été conclu au regard des résultats obtenus dans l'exemple 3 ci-dessus, la quantité optimale en protéine TRAF2, permettant d'obtenir une détection très sensible de la présence de la protéine virale de capside du VHC dans un sérum humain (dès 6549 UI/ml) tout en limitant l'obtention de faux positifs (intensité du spot différent de 0 % du signal maximal en l'absence de protéine virale de capside du VHC), est de 25 ng/µl.

## Revendications

1. Utilisation, *in vitro* ou *ex vivo,* d'au moins une protéine Facteur 2 associée au récepteur du Facteur de nécrose tumorale, TRAF2, ou d'un homologue de TRAF2, comme agent de détection d'une protéine virale de capside du Virus de l'Hépatite C, p21, l'homologue de TRAF2 présentant un degré d'homologie d'au moins 85% avec la protéine TRAF2 et une aptitude similaire à lier une protéine virale de capside p21 du Virus de l'Hépatite C.

2. Utilisation selon la revendication 1, dans laquelle la protéine TRAF2 est une protéine TRAF2 humaine.

3. Procédé, *in vitro* ou *ex vivo,* pour détecter la présence présumée d'une protéine virale de capside du Virus de l'Hépatite C, p21, dans un échantillon biologique, ledit procédé comprenant au moins les étapes consistant à :
a- mettre en contact ledit échantillon avec une quantité efficace d'au moins une protéine Facteur 2 associée au récepteur du Facteur de nécrose tumorale, TRAF2, ou d'un homologue de TRAF2, dans des conditions appropriées à la formation d'au moins un complexe protéique entre la protéine TRAF2 ou l'homologue de TRAF2, et au moins une protéine virale de capside du Virus de l'Hépatite C, p21,
b- mettre en contact le complexe protéique de l'étape a- avec une entité bi-fonctionnelle, ladite entité étant dotée d'un groupement apte à se lier spécifiquement au moins à une protéine virale de capside p21, dans ledit complexe, et d'un groupement apte à être détectable, directement ou indirectement, par un mode de détection, la mise en contact étant effectuée dans des conditions appropriées à l'établissement d'une liaison entre ladite protéine virale de capside p21 et ladite entité bi-fonctionnelle,
c- détecter ladite entité bi-fonctionnelle, la détection de ladite entité bi-fonctionnelle liée étant indicatrice de la présence de la protéine virale de capside p21,
l'homologue de TRAF2 présentant un degré d'homologie d'au moins 85% avec la protéine TRAF2 et une aptitude similaire à lier une protéine virale de capside p21 du Virus de l'Hépatite C.

4. Procédé selon la revendication précédente, comprenant au moins une étape supplémentaire après l'étape b- et avant l'étape c-, ladite étape supplémentaire consistant à éliminer l'entité bi-fonctionnelle non liée à la protéine virale de capside p21.

5. Procédé selon la revendication précédente, dans lequel la protéine TRAF2, ou son homologue, est immobilisé(e) sur un support organique ou inorganique.

6. Procédé selon la revendication précédente, dans lequel le support est constitué d'un matériau de cellulose, de nitrocellulose, en plastique, en verre, en céramique, en silice, ou en résine polymérique.

7. Procédé selon l'une quelconque des revendications 3 à 6, dans lequel ladite entité bi-fonctionnelle est un anticorps ou un fragment d'anticorps.

8. Procédé selon l'une quelconque des revendications 3 à 7, dans lequel ledit groupement détectable est un groupement propice à une détection luminescente, colorimétrique, ou radioactive, de préférence colorimétrique.

9. Kit de détection d'au moins une protéine virale de capside du Virus de l'Hépatite C, p21, comprenant :
- au moins un premier réceptacle comprenant au moins une protéine Facteur 2 associée au récepteur du Facteur de nécrose tumorale, TRAF2, ou un homologue de TRAF2, et
- au moins un second réceptacle comprenant une entité bi-fonctionnelle dotée d'un groupement apte à se lier spécifiquement au moins à ladite protéine virale de capside p21, susceptible d'être présent(e) dans un complexe protéique formé avec la protéine TRAF2, ou l'homologue de TRAF2, et un groupement apte à être détectable, directement ou indirectement, par un mode de détection,
l'homologue de TRAF2 présentant un degré d'homologie d'au moins 85% avec la protéine TRAF2 et une aptitude similaire à lier une protéine virale de capside p21 du Virus de l'Hépatite C.

10. Complexe protéique isolé, constitué d'une protéine Facteur 2 associée au récepteur du Facteur de nécrose tumorale, TRAF2, ou un homologue de TRAF2, et d'une protéine virale de capside du Virus de l'Hépatite C, p21, l'homologue de TRAF2 présentant un degré d'homologie d'au moins 85% avec la protéine TRAF2 et une aptitude similaire à lier une protéine virale de capside p21 du Virus de l'Hépatite C.

11. Complexe protéique constitué d'une protéine Facteur 2 associée au récepteur du Facteur de nécrose tumorale, TRAF2, ou un homologue de TRAF2, et d'une protéine virale de capside du Virus de l'Hépatite C, p21, ledit complexe étant immobilisé sur un support, l'homologue de TRAF2 présentant un degré d'homologie d'au moins 85% avec la protéine TRAF2 et une aptitude similaire à lier une protéine virale de capside p21 du Virus de l'Hépatite C..

## Patentansprüche

1. Verwendung, *in vitro* oder ex *vivo,* mindestens eines Tumornekrosefaktor-Rezeptor assoziierter Faktor 2-Proteins, TRAF2, oder eines Homologons von TRAF2 als Mittel zum Nachweis eines viralen Capsidproteins des Hepatitis C-Virus, p21, wobei das Homologon von TRAF2 einen Grad der Homologie von mindestens 85% mit dem TRAF2-Protein und eine ähnliche Fähigkeit, ein virales Capsidprotein p21 des Hepatitis C-Virus zu binden, aufweist.

2. Verwendung nach Anspruch 1, wobei es sich bei dem TRAF2-Protein um ein menschliches TRAF2-Protein handelt.

3. *In vitro-* oder *Ex* vivo-Verfahren zum Nachweisen des vermuteten Vorhandenseins eines viralen Capsidproteins des Hepatitis C-Virus, p21, in einer biologischen Probe, wobei das Verfahren mindestens die folgenden Schritte umfasst:
a- Inkontaktbringen der Probe mit einer wirksamen Menge mindestens eines Tumornekrosefaktor-Rezeptor assoziierter Faktor 2-Proteins, TRAF2, oder eines Homologons von TRAF2 unter Bedingungen, die zur Bildung mindestens eines Proteinkomplexes zwischen dem TRAF2-Protein oder dem Homologon von TRAF2 und mindestens einem viralen Capsidprotein des Hepatitis C-Virus, p21, geeignet sind,
b- Inkontaktbringen des Proteinkomplexes aus Schritt a- mit einer bifunktionellen Einheit, wobei diese Einheit versehen ist mit einer Gruppe, die in der Lage ist, spezifisch an mindestens an ein virales Capsidprotein p21 in dem Komplex zu binden, und einer Gruppe, die direkt oder indirekt durch ein Nachweisverfahren nachgewiesen werden kann, wobei das Inkontaktbringen unter Bedingungen durchgeführt wird, die zur Herstellung einer Bindung zwischen dem viralen Capsidprotein p21 und der bifunktionellen Einheit geeignet sind.
c- Nachweisen der bifunktionellen Einheit, wobei der Nachweis der gebundenen bifunktionellen Einheit ein Hinweis auf das Vorhandensein des viralen Capsidproteins p21 ist,
wobei das Homologon von TRAF2 einen Grad der Homologie von mindestens 85% mit dem TRAF2-Protein und eine ähnliche Fähigkeit, ein virales Capsidprotein p21 des Hepatitis C-Virus zu binden, aufweist.

4. Verfahren nach dem vorhergehenden Anspruch, das mindestens einen zusätzlichen Schritt nach Schritt bund vor Schritt c- umfasst, wobei man in dem zusätzlichen Schritt die nicht an das virale Capsidprotein p21 gebundene bifunktionelle Einheit entfernt.

5. Verfahren nach dem vorhergehenden Anspruch, wobei das TRAF2-Protein oder dessen Homologon auf einem organischen oder anorganischen Träger immobilisiert ist.

6. Verfahren nach dem vorhergehenden Anspruch, wobei der Träger aus einem Cellulose-, Nitrocellulose-, Kunststoff-, Glas-, Keramik-, Siliziumdioxidmaterial oder aus Polymerharz besteht.

7. Verfahren nach einem der Ansprüche 3 bis 6, wobei die bifunktionelle Einheit ein Antikörper oder ein Antikörperfragment ist.

8. Verfahren nach einem der Ansprüche 3 bis 7, wobei es sich bei der nachweisbaren Gruppe um eine Gruppe handelt, die für einen Lumineszenz-, kolorimetrischen oder radioaktiven, vorzugsweise kolorimetrischen, Nachweis geeignet ist.

9. Kit zum Nachweis mindestens eines viralen Capsidproteins des Hepatitis C-Virus, p21, umfassend:
- mindestens einen ersten Behälter, der mindestens ein Tumornekrosefaktor-Rezeptor assoziierter Faktor 2-Protein, TRAF2, oder ein Homologon von TRAF2 umfasst, und
- mindestens einen zweiten Behälter, der eine bifunktionelle Einheit umfasst, die versehen ist mit einer Gruppe, die fähig ist, spezifisch an mindestens das virale Capsidprotein p21 zu binden, das möglicherweise in einem mit dem TRAF2-Protein oder dem Homologon von TRAF2 gebildeten Proteinkomplex vorhanden ist, und einer Gruppe, die direkt oder indirekt durch ein Nachweisverfahren nachgewiesen werden kann,
wobei das Homologon von TRAF2 einen Grad der Homologie von mindestens 85% mit dem TRAF2-Protein und eine ähnliche Fähigkeit, ein virales Capsidprotein p21 des Hepatitis C-Virus zu binden, aufweist.

10. Isolierter Proteinkomplex, der aus einem Tumornekrosefaktor-Rezeptor assoziierter Faktor 2-Protein, TRAF2, oder einem Homologon von TRAF2 und einem viralen Capsidprotein des Hepatitis C-Virus, p21, besteht, wobei das Homologon von TRAF2 einen Grad der Homologie von mindestens 85% mit dem TRAF2-Protein und eine ähnliche Fähigkeit, ein virales Capsidprotein p21 des Hepatitis C-Virus zu binden, aufweist.

11. Proteinkomplex, der aus einem Tumornekrosefaktor-Rezeptor assoziierter Faktor 2-Protein, TRAF2, oder einem Homologon von TRAF2 und einem viralen Capsidprotein des Hepatitis C-Virus, p21, besteht, wobei der Komplex auf einem Träger immobilisiert ist, wobei das Homologon von TRAF2 einen Grad der Homologie von mindestens 85% mit dem TRAF2-Protein und eine ähnliche Fähigkeit, ein virales Capsidprotein p21 des Hepatitis C-Virus zu binden, aufweist.

## Claims

1. Use, *in vitro* or ex *vivo,* of at least one tumour necrosis factor receptor-associated factor 2 protein, TRAF2, or a TRAF2 homologue, as agent for detecting a viral capsid protein of hepatitis C virus, p21, the TRAF2 homologue having a degree of homology of at least 85% with the TRAF2 protein and a similar ability to bind a viral capsid protein p21 of hepatitis C virus.

2. Use according to Claim 1, in which the TRAF2 protein is a human TRAF2 protein.

3. Process, *in vitro* or ex *vivo,* for detecting the presumed presence of a viral capsid protein of hepatitis C virus, p21, in a biological sample, said process comprising at least the steps consisting in:
a- placing said sample in contact with an effective amount of at least one tumour necrosis factor receptor-associated factor 2 protein, TRAF2, or a TRAF2 homologue, under suitable conditions for the formation of at least one protein complex between the TRAF2 protein or the TRAF2 homologue and at least one viral capsid protein of hepatitis C virus, p21,
b- placing the protein complex from step a- in contact with a bifunctional entity, said entity being provided with a group able to specifically bind at least to a viral capsid protein p21, in said complex, and a group able to be detected directly or indirectly by a detection method, the contacting being carried out under suitable conditions for establishing a connection between said viral capsid protein p21 and said bifunctional entity,
c- detecting said bifunctional entity, the detection of said bound bifunctional entity being indicative of the presence of the viral capsid protein p21,
the TRAF2 homologue having a degree of homology of at least 85% with the TRAF2 protein and a similar ability to bind a viral capsid protein p21 of hepatitis C virus.

4. Process according to the preceding claim, comprising at least one additional step after step b- and before step c-, said additional step consisting in eliminating the bifunctional entity not bound to the viral capsid protein p21.

5. Process according to the preceding claim, wherein the TRAF2 protein, or the homologue thereof, is immobilized on an organic or inorganic support.

6. Process according to the preceding claim, wherein the support consists of a cellulose, nitrocellulose, plastic, glass, ceramic, silica or polymer resin material.

7. Process according to any one of Claims 3 to 6, wherein said bifunctional entity is an antibody or an antibody fragment.

8. Process according to any one of Claims 3 to 7, wherein said detectable group is an appropriate group for luminescent, colorimetric or radioactive detection, preferably colorimetric detection.

9. Kit for detecting at least one viral capsid protein of hepatitis C virus, p21, comprising:
- at least one first container comprising at least one tumour necrosis factor receptor-associated factor 2 protein, TRAF2, or a TRAF2 homologue, and
- at least one second container comprising a bifunctional entity provided with a group able to specifically bind at least to said viral capsid protein p21, liable to be present in a protein complex formed with the TRAF2 protein, or TRAF2 homologue, and a group able to be detected directly or indirectly by a detection method,
the TRAF2 homologue having a degree of homology of at least 85% with the TRAF2 protein and a similar ability to bind a viral capsid protein p21 of hepatitis C virus.

10. Isolated protein complex, consisting of a tumour necrosis factor receptor-associated factor 2 protein, TRAF2, or a TRAF2 homologue, and a viral capsid protein of hepatitis C virus, p21, the TRAF2 homologue having a degree of homology of at least 85% with the TRAF2 protein and a similar ability to bind a viral capsid protein p21 of hepatitis C virus.

11. Protein complex consisting of a tumour necrosis factor receptor-associated factor 2 protein, TRAF2, or a TRAF2 homologue, and a viral capsid protein of hepatitis C virus, p21, said complex being immobilized on a support, the TRAF2 homologue having a degree of homology of at least 85% with the TRAF2 protein and a similar ability to bind a viral capsid protein p21 of hepatitis C virus.
